# EUROPEAN PATENT APPLICATION

(11) **EP 2 505 190 A1**
(43) Date of publication of application: **03.10.2012**
(21) Application number: 12172595.6
(22) Date of filing: 12.06.2009
(51) Int. Cl.: A61K 9/00, A61K 31/407, C07D 477/20, A61K 9/19

(54) **Polymorphic forms of ertapenem monosodium salt and process for it's preparation**

(30) Priority: 11.06.2008 IN DE13852008
(62) Divisional of application: 09762157.7
(71) Applicant: Ranbaxy Laboratories Limited, New Delhi 110019 (IN)
(72) Inventor: George, Vinod, 679333 Kerala (IN); Vashishta, Bhupendra, 160047 Chandigarh (IN); Prasad, Mohan, 122002 Haryana (IN); Kumar, Naresh, 122018 Haryana (IN); Arora, Vinod, Kumar, 122101 Haryana (IN)
(74) Representative: Cronin, Brian Harold John

(57) **Abstract**

The invention relates to a polymorphic form of ertapenem monosodium (Formula I) as Form D and its preparation. 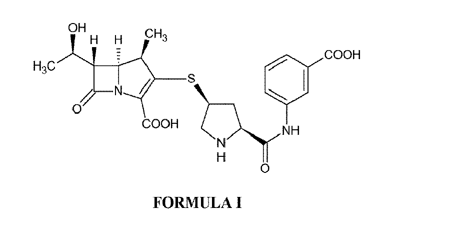 Polymorphic form D is characterized by having an XRPD pattern showing 2 theta values at 4.44±0.2, 5.26±0.2, 7.44±0.2, 8.12±0.2, 10.98±0.2, 12.74±0.2, 19.28±0.2, 22.93±0.2, 23.51±0.2, 25.07±0.2 and 30.15 ±0.2.

## Description

### Field of the Invention

The present invention relates to process for preparing a carbapenem antibiotic composition. The present invention further relates to a carbapenem antibiotic composition substantially free of degradation impurities. The present invention further relates to a polymorphic form of ertapenem monosodium designated as Form D and its preparation.

### Background of the Invention

Carbapenem antibiotics are widely used in treating infections caused by broad range of pathogens including multi-drug-resistant bacteria. Ertapenem sodium is a carbapenem antibiotic, which is chemically a monosodium or a di-sodium salt of 4*R-*[3(3*S**,5*S**),4α,5β,6β(*R**)]]-3-[[5-[[(3-carboxyphenyl)amino]carbonyl]-3-pyrrolidinyl ]thio]-6-(1-hydroxyethyl)-4-methyl-7-oxo-1- azabicyclo[3.2.0]hept-2-ene-2-carboxylic acid of Formula I.

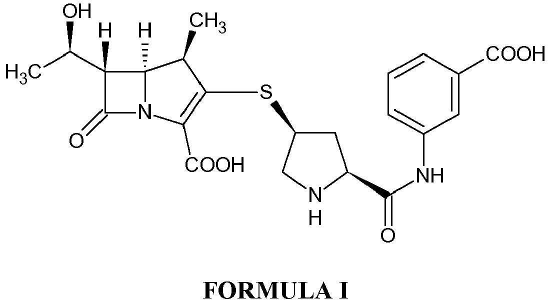

A process for the preparation of ertapenem sodium is provided in U.S. Patent No. 5,652,233 and J. Org. Chem. (2005) 70:7479-7487. U.S. Patent No. 7,022,841 provides a process for preparing ertapenem sodium as crystalline Form A, Form B and Form C. Form A is obtained by crystallizing ertapenem sodium from a mixture of water, methanol and 1-propanol. When Form A is washed with a mixture of 2-propanol and water (85:15 v/v), Form B is obtained. When Form B is washed with methyl acetate containing 2% w/v of water, Form C is obtained. U.S. Patent No. 7,022,841 also provides a process for reducing residual solvents from Form A, Form B and Form C by sweeping nitrogen through the wet solid at low temperature conditions and by employing agitated filter dryers.

U.S. Patent No. 6,548,492 says that ertapenem sodium is sensitive to temperature and pH fluctuations. According to U.S. Patent No. 6,548,492, ertapenem sodium remains unstable at temperatures above about -20°C. U.S. Patent No. 6,548,492 further says that ertapenem sodium undergoes dimerization and hydrolysis to form undesirable dimers and ring-opened compounds above about -20°C. J. Liq. Chrom. & Rel. Technol., (2001) 24(19):2999-3015 provides the preparation, isolation and characterization data of the degradation impurities of ertapenem sodium, which are referred as Dimer I, Dimer II, Dimer III, Dimer-H₂Oa, Dimer-H₂Ob, Dimer V and 'Ring Opened'.

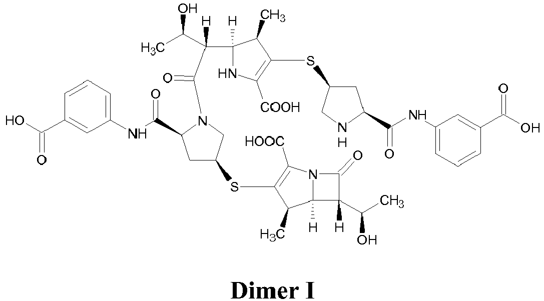

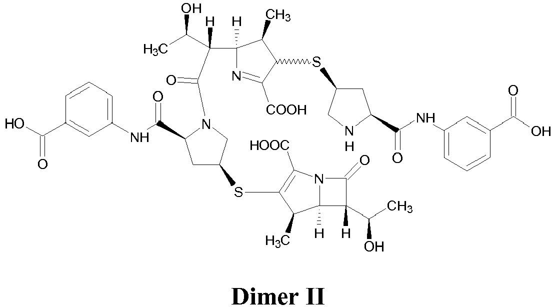

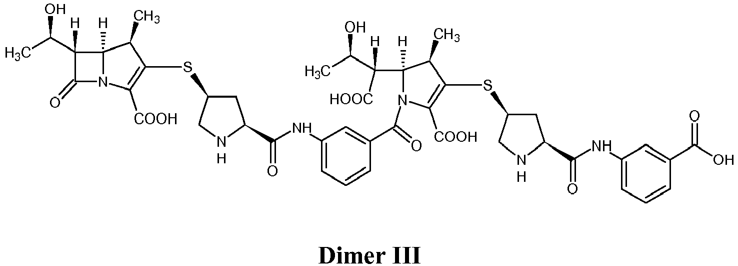

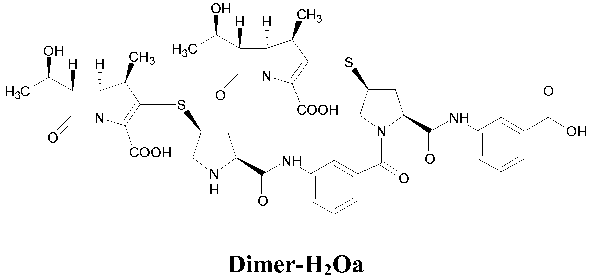

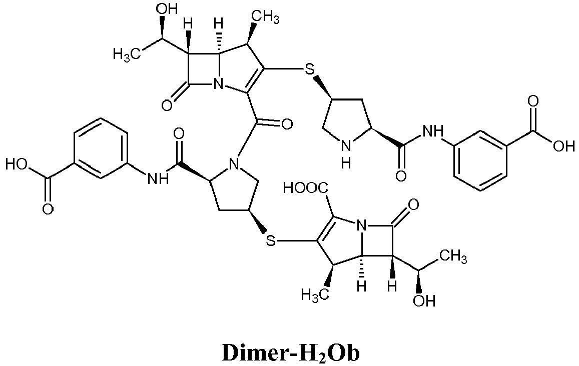

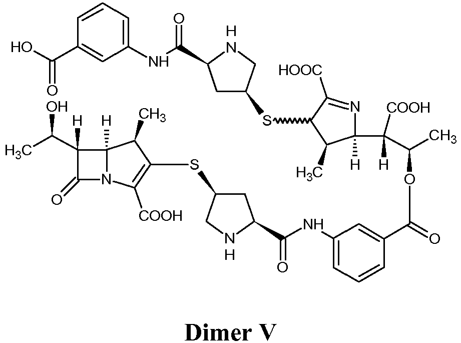

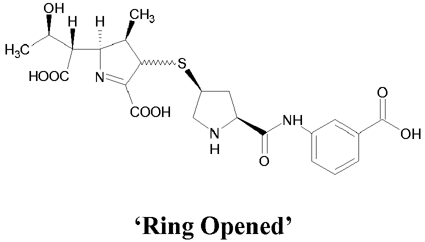

As mentioned in U.S. Patent No. 5,952,323, when ertapenem or its sodium salts are formulated in a pharmaceutical composition with a suitable amount of sodium carbonate or sodium bicarbonate, the compound of Formula II or its salts are formed upon dilution or reconstitution.

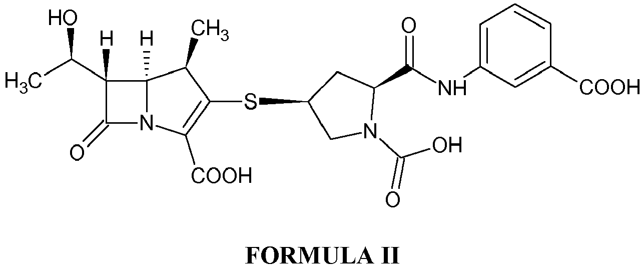

The compound of Formula II or its salts are more stable than ertapenem sodium and the formation of degradation impurities is minimized. Therefore, ertapenem sodium is commercially available as a pharmaceutical composition comprising the compound of Formula II or its salts for parenteral administration. U.S. Patent No. 6,548,492 describes a vial lyophilization process for the preparation of a pharmaceutical composition comprising the compound of Formula II or its salts. According to said process, a sterile aqueous solution containing the compound of Formula II or its salts is prepared, filled in vials and lyophilized under specific lyophilization conditions, and the vials are removed as final formulation. The lyophilization method involves a secondary drying phase of heating the composition up to about 60°C at a pressure of 80 mTorr.

However, the lyophilized product obtained according to U.S. Patent No. 6,548,492 process has a purity of only about 95% and it contains more than 2% of ring opening impurities. Further, the dimer impurities are present in the lyophilized product in a range of 1.5% to 1.6% in lab scale batch and it is increased more than 2% in pilot plant scale batch.

The present inventors have further observed that the drying processes of ertapenem sodium described in U.S. Patent No. 7,022,841 using nitrogen gas and agitated filter dryers also cause the generation of degradation impurities. Conversion of such impure ertapenem sodium into a pharmaceutical composition comprising the compound of Formula II or its salts further increases the content of degradation impurities in the final formulation.

### Summary of the Invention

The present inventors have developed an advantageous process for the preparation of a pharmaceutical composition comprising the compound of Formula II or its salts. The present process involves simple tray lyophilization, wherein the secondary drying is carried out at a temperature of about 30°C or below. The pharmaceutical composition obtained by the process of present invention has a purity of about 97% or more and it is substantially free of Dimer I, Dimer II, Dimer III, Dimer-H₂O Dimer-H₂O Dimer V and 'Ring Opened' impurities. The present invention also provides polymorphic Form D of ertapenem monosodium, which is significantly different from the polymorphic forms of ertapenem sodium described in the prior art. The novel polymorphic Form D of the present invention is stable and it can be easily formulated into a pharmaceutical composition comprising the compound of Formula II or its salts. The present inventors have also developed an efficient process for the purification of ertapenem sodium, wherein the process avoids vigorous drying methods using nitrogen sweep or agitation and thereby minimizes the formation of degradation impurities. Thus, the present invention provides an advantageous, efficient and industrially preferable process for the preparation of a pharmaceutical composition comprising the compound of Formula II or its salts.

### Brief Description of the Drawings

Figure 1 depicts the X-Ray Powder Diffractogram (XRPD) of the pharmaceutical composition obtained according to Example 2.
Figure 2 depicts the X-Ray Powder Diffractogram (XRPD) of polymorphic Form D of ertapenem sodium.

### Detailed Description of the Invention

A first aspect of the present invention provides polymorphic Form D of ertapenem monosodium of Formula Ia.

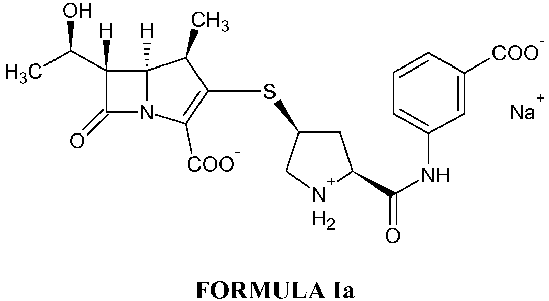

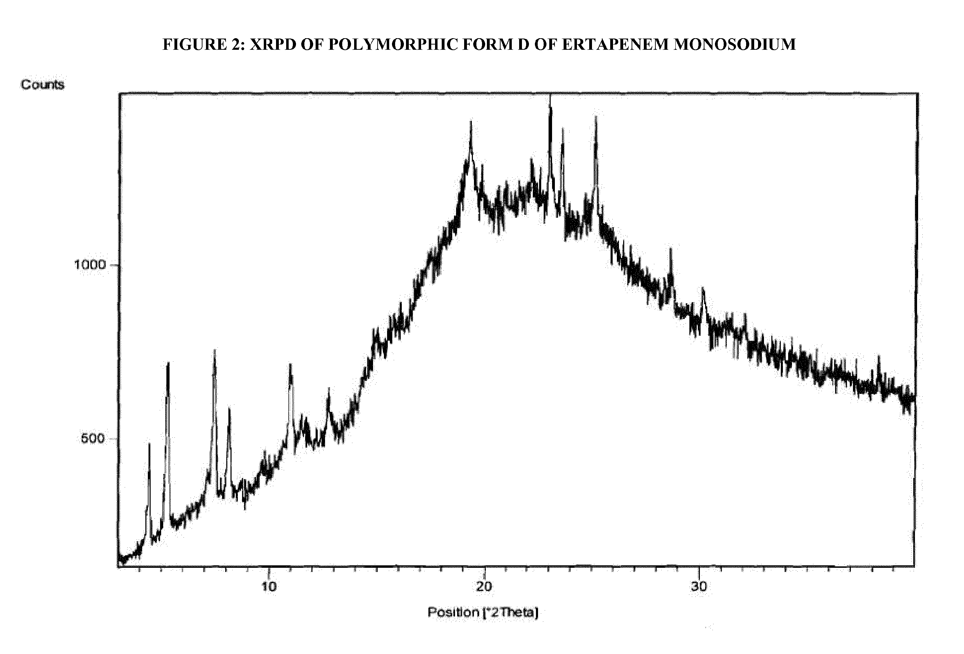

The polymorphic Form D is characterized by substantially the same XRPD pattern as depicted in Figure 2 of the accompanied drawing. The XRPD pattern of polymorphic Form D shows characteristic 2 theta values at 4.44±0.2, 5.26±0.2, 7.44±0.2, 8.12±0.2, 10.98±0.2, 12.74±0.2, 19.28±0.2, 22.93±0.2, 23.51±0.2, 25.07±0.2 and 30.15±0.2.

A second aspect of the present invention provides a process for the preparation of polymorphic Form D of ertapenem monosodium of Formula Ia, 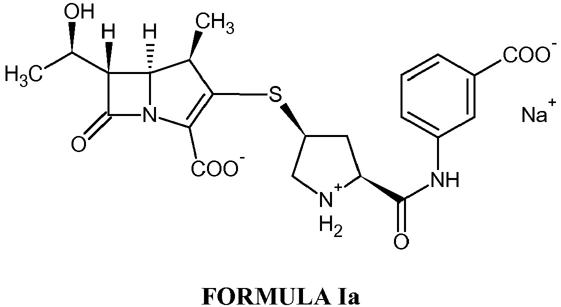 wherein the process comprises,
a) treating ertapenem monosodium of Formula Ia with water and methanol to obtain a solution,
b) treating the solution obtained in step a) with n-propanol,
c) stirring the mixture obtained in step b) at a temperature of about 0°C or below to obtain a solid, and
d) treating the solid obtained in step c) with acetone to obtain polymorphic Form D of ertapenem monosodium of Formula Ia.

Ertapenem monosodium existing in any solid form known in the art may be used as a starting material. Ertapenem monosodium is treated with water and methanol to obtain a solution. The treatment may be carried out in the presence of a base and the pH of the solution may be subsequently adjusted to about 5 to about 6, for example, to about 5.5 5 to about 5.7 by treating with an organic or inorganic acid. The base may be an alkali metal carbonate or alkali metal bicarbonate, for example, sodium bicarbonate or sodium carbonate. The acid may be an organic or inorganic acid, for example, acetic acid. The solution obtained may be cooled to about 5°C or below and treated with n-propanol. The n-propanol may also be in the form of a mixture with methanol. The mixture obtained is stirred at a temperature of about 0°C or below, for example, at about -20° to about 0°C for about 10 minutes to about 100 h to obtain a solid. The solid is isolated from the mixture, for example, by filtration, treated with acetone and dried to obtain polymorphic Form D of ertapenem monosodium. The polymorphic Form D of ertapenem monosodium may be further converted into a pharmaceutical composition comprising the compound of Formula II or its salts by the methods known in the prior art or according to the methods disclosed in the present application.

A third aspect of the present invention provides a process for the purification of ertapenem sodium, wherein the process comprises,
a) dissolving crude ertapenem sodium in water in the presence of a base,
b) adjusting the pH of the solution obtained in step a) to about 5 to about 6,
c) treating the solution obtained in step b) with one or more alkanols, and
d) isolating the pure ertapenem sodium from the mixture thereof.

Crude ertapenem sodium used as a starting material may contain process related and/or degradation impurities in a quantity not preferable for pharmaceutical use, for example in a quantity of about 4% or more. Crude ertapenem sodium may be obtained during the process of preparing ertapenem sodium according to the prior art, for example, U.S. Patent Nos. 5,652,233 and 7,022,841, and J. Org. Chem. (2005) 70:7479-7487, or during the storage of ertapenem sodium obtained by prior art processes. Crude ertapenem sodium is dissolved in water in the presence of a base at a temperature of about 0° to about 10°C. The base may be an alkali metal carbonate or alkali metal bicarbonate, for example, sodium bicarbonate or sodium carbonate. The solution may be optionally further diluted with an alkanol, for example, methanol. The pH of the solution is adjusted to about 5 to about 6, for example, to about 5.5 to about 5.7 at a temperature of about 0° to about 10°C. The pH adjustment may be carried out by treating with an organic or inorganic acid, for example, acetic acid. The solution so obtained may be treated with activated carbon and filtered. The solution is treated with one or more alkanols. The alkanol may be a C₃₋₆ alcohol, for example, n-propanol, or a mixture of a C₃₋₆ alcohol with methanol. The mixture obtained is stirred at about -20° to about 0°C for about 10 minutes to about 100 h. The solid is isolated from the mixture, for example by filtration, and optionally washed with a ketone, for example, acetone, to obtain pure ertapenem sodium. The ertapenem sodium may be further converted into a pharmaceutical composition comprising the compound of Formula II or its salts by the methods known in the prior art or according to the methods disclosed in the present application.

A fourth aspect of the present invention provides a pharmaceutical composition comprising the compound of Formula II or its salts, 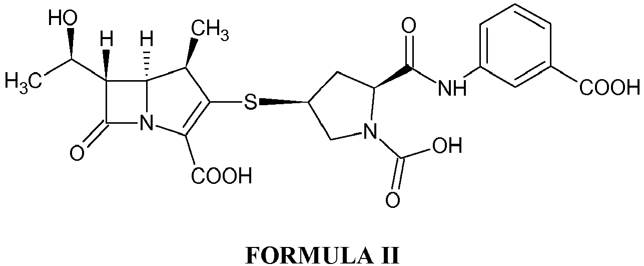 wherein the pharmaceutical composition has a purity of about 97% or more. The compounds of Formula IIa through IIg are examples of the salt forms of the compound of Formula II. 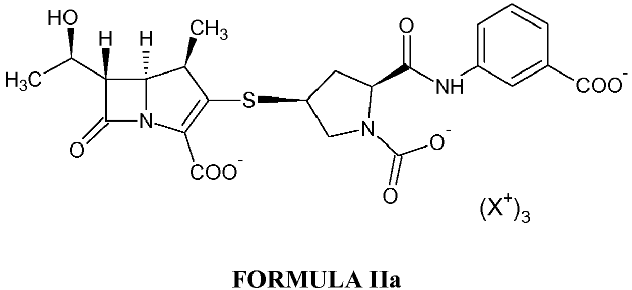 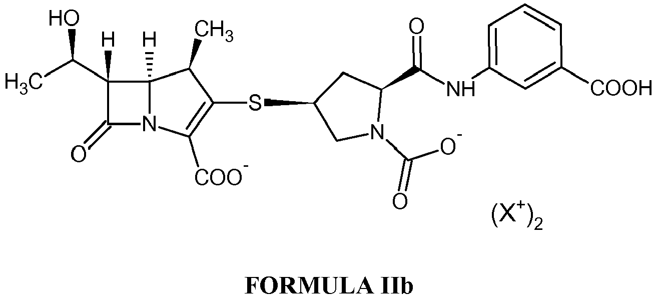 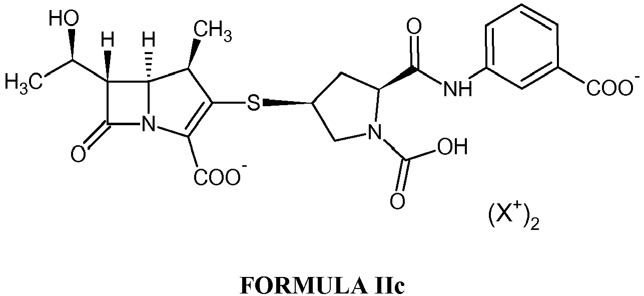 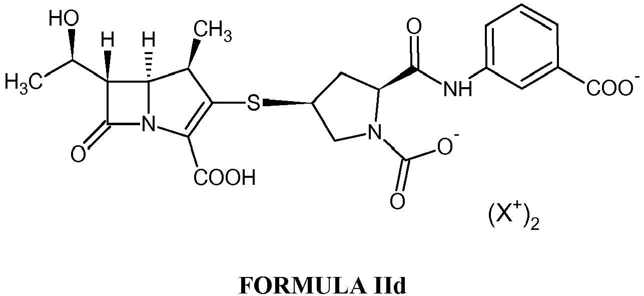 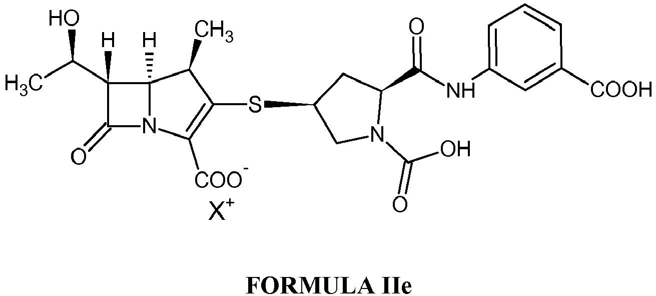 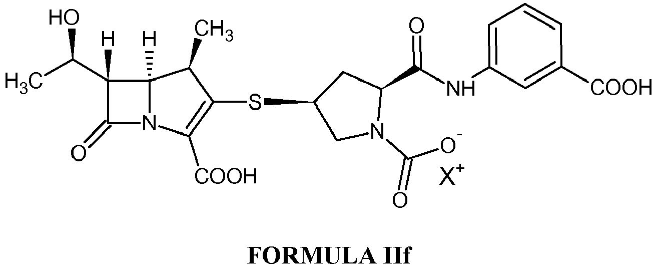 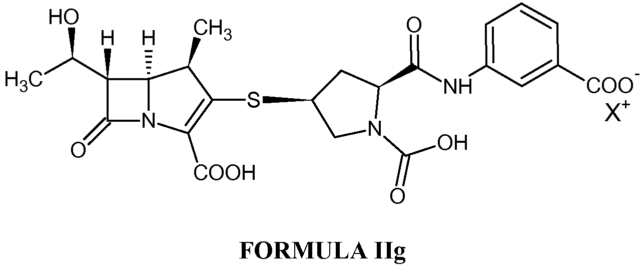 wherein X is sodium or potassium. The pharmaceutical composition of the present invention comprises the compound of Formula II preferably as a sodium salt.

The pharmaceutical composition of the present invention is substantially free of Dimer I of the following formula.

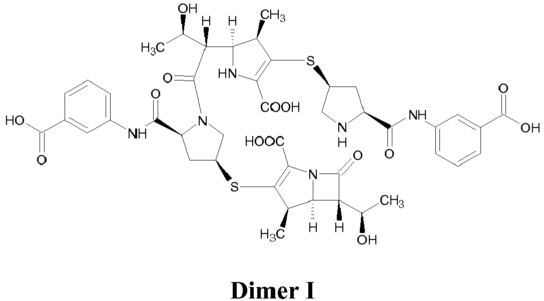

Dimer I is present in the pharmaceutical composition of the present invention in an amount of about 0.75% or below.

The pharmaceutical composition of the present invention is substantially free of Dimer II of the following formula.

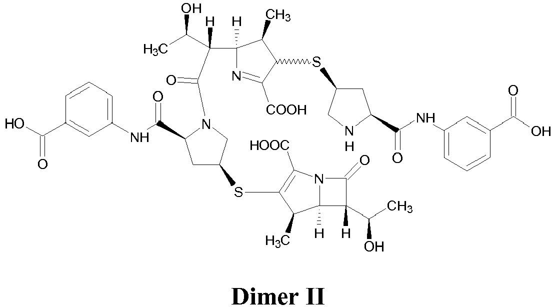

Dimer II is present in the pharmaceutical composition of the present invention in an amount of about 0.75% or below.

The pharmaceutical composition of the present invention is substantially free of Dimer III of the following formula.

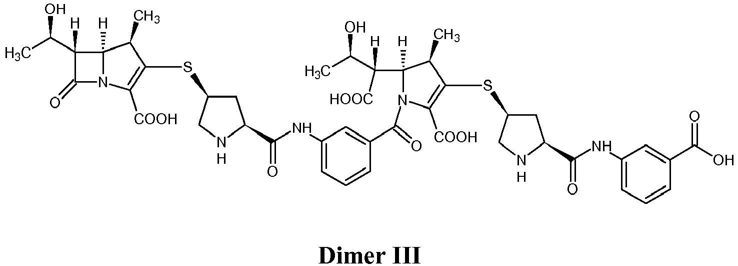

Dimer III is present in the pharmaceutical composition of the present invention in an amount of about 0.20% or below.

The pharmaceutical composition of the present invention is substantially free of Dimer H₂Oa of the following formula.

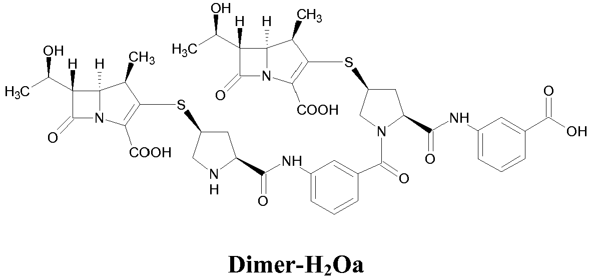

Dimer H₂Oa is present in the pharmaceutical composition of the present invention in an amount of about 0.50% or below.

The pharmaceutical composition of the present invention is substantially free of Dimer H₂Ob of the following formula.

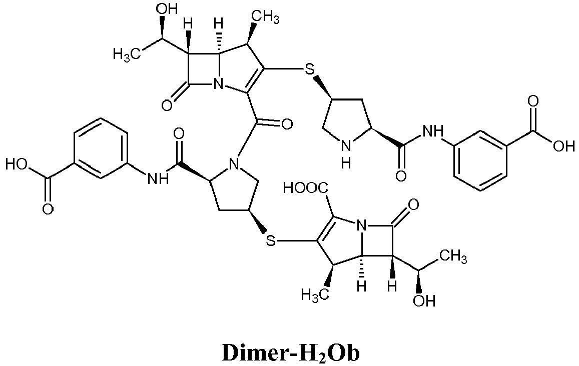

Dimer H₂Ob is present in the pharmaceutical composition of the present invention in an amount of about 0.50% or below.

The pharmaceutical composition of the present invention is substantially free of Dimer V of the following formula.

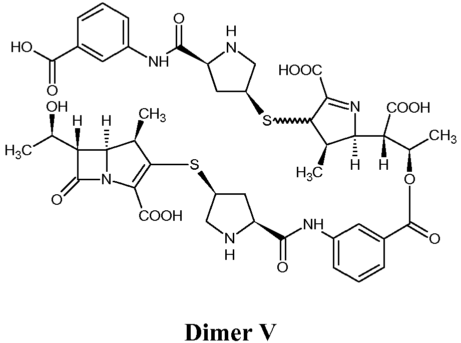

Dimer V is present in the pharmaceutical composition of the present invention in an amount of about 0.10% or below.

The pharmaceutical composition of the present invention is substantially free of Ring Opened impurity of the following formula.

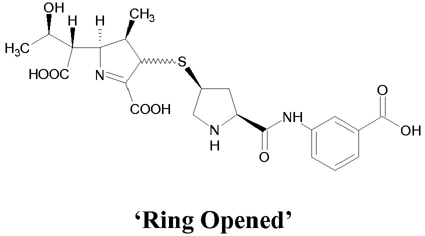

Ring Opened impurity is present in the pharmaceutical composition of the present invention in an amount of about 1.0 % or below.

The pharmaceutical composition of the present invention further comprises one or more pharmaceutical excipients including diluents, buffers, preservatives, local anesthetics and tonicity controlling agents. The diluents include sterile water for injection, normal saline, dextrose solution, Ringer's solution and the like. The buffers include dihydrogen sodium phosphate, citrate buffer, meglumine, tri(hydroxymethyl)aminomethane, and the like. The preservative include butylhydroxyacetone, butylhydroxytoluene, benzalkonium chloride and the like. Local anesthetics include benzocaine, lidocaine, novacaine, pontocaine and the like. Tonicity modifying agents include sodium chloride, mannitol, dextrose, glucose, lactose, sucrose and the like.

A fifth aspect of the present invention provides a process for the preparation of a pharmaceutical composition comprising the compound of Formula II or its salts, and having a purity of about 97% or more, 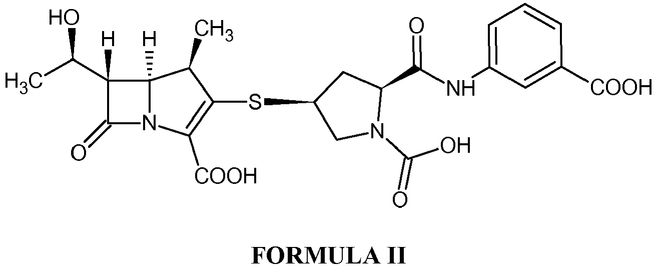 wherein the process comprises,
a) adding ertapenem sodium to an aqueous solution containing a carbon dioxide producing compound, or adding an aqueous solution containing a carbon dioxide producing compound to ertapenem sodium while maintaining the pH between about 7 to about 8, to obtain a solution comprising the compound of Formula II or its salts,
b) lyophilizing the solution obtained in step a) on a lyophilization tray, and
c) unloading the pharmaceutical composition comprising the compound of Formula II or its salts, and having a purity of about 97% or more, from the lyophilization tray.

Ertapenem sodium used as the starting material may be prepared according to the methods provided in the previous aspects of the present invention. Ertapenem sodium is added to an aqueous solution containing a carbon dioxide producing compound, or an aqueous solution containing a carbon dioxide producing compound is added to ertapenem sodium while maintaining the pH between about 7 to about 8 to obtain a solution comprising the compound of Formula II or its salts. The pH may be maintained, for example, between about 7.4 to about 7.7 during said addition. The pH may be maintained in said range by a sequential addition of a base. The base may be, for example, sodium hydroxide. The addition may be carried out at a temperature of about 0° to about 10°C, for example, about 0°C to about 5°C. The carbon dioxide producing compound may be selected from the group consisting of alkali metal carbonates, alkali metal bicarbonates, alkaline earth metal carbonates and alkaline earth metal bicarbonates. The carbon dioxide producing compound may be, for example, sodium bicarbonate or sodium carbonate. The solution so obtained is optionally filtered through about 0.2 micron membrane filter to obtain a sterile solution. The solution is subsequently lyophilized on a lyophilization tray. Conventional lyophilization trays including steel, aluminum, glass, plastic or Lyoguard^{®} trays may be used. Total mass and tray thickness may be suitably optimized. The lyophilization process may be carried out by freezing the solution comprising the compound of Formula II or its salts at a temperature of about -40°C or below for about 2 to about 4 h to obtain a frozen composition. The primary drying of the frozen composition may be carried out at a temperature of about -25°C to about 0°C for about 2 to about 20 h. The secondary drying may be carried out at a temperature of about 30°C or below, for example, at about 10°C to about 30°C. The secondary drying may be carried out for about 1 to about 40 h. The entire drying process may be carried out under pressure of about 100 mTorr or below, for example, about 85 to about 100 mTorr. The lyophilized pharmaceutical composition comprising the compound of Formula II or its salts, for example, a sodium salt, and having a purity of about 97% or more, is unloaded from the lyophilization tray. The pharmaceutical composition so obtained may be further mixed with one or more pharmaceutical excipients and packed suitably in containers including, for example, vials and ampoules.

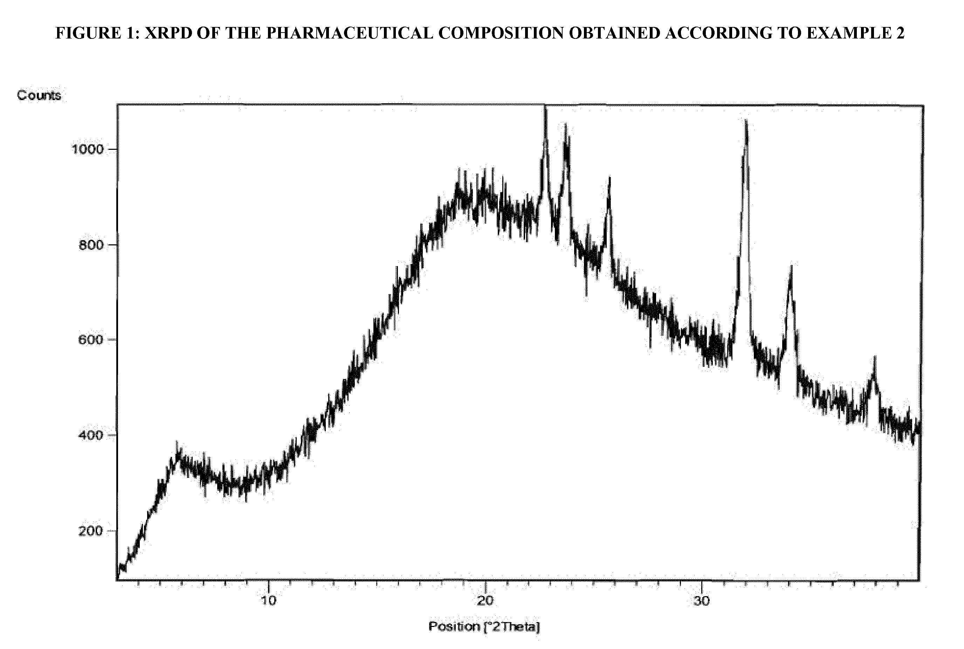

A sixth aspect of the present invention provides a pharmaceutical composition comprising a sodium salt of the compound of Formula II, 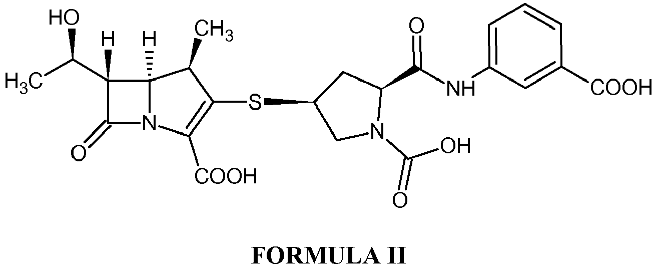 wherein the pharmaceutical composition has substantially the same XRPD pattern as depicted in Figure 1 of the accompanied drawing. The XRPD of the pharmaceutical composition has characteristic 2 theta values at 22.63±0.2, 23.50±0.2, 25.54±0.2, 31.86±0.2, 33.95±0.2 and 37.79±0.2. The pharmaceutical composition is further characterized by a sodium content of about 10.5% to about 12.0%, for example, about 11.0%.

A seventh aspect of the present invention provides a method of treating a bacterial infection comprising administering to a patient in need thereof an antibacterially effective amount of a pharmaceutical composition comprising the compound of Formula II or its salts, 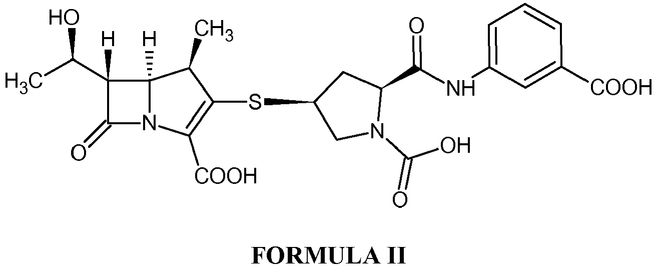 wherein the pharmaceutical composition has a purity of about 97% or more.

The purity was determined by high-performance liquid chromatography (HPLC) method using ACE C 8, 5µ column and UV detector (250 nm). Gradient system Acetonitrile: Buffer (Ammonium acetate/ Acetic acid pH 6.5) was used as a mobile phase and the flow rate was 1.0 ml/minute.

XRPD of the samples were determined by using Panalytical X'Pert Pro X-Ray Powder Diffractometer in the range 3 to 40 degree 2 theta with a step size of 0.02 and under tube voltage and current of 45 Kv and 40 mA respectively. Copper radiation of wavelength 1.54 angstrom and Xceletor detector were used.

While the present invention has been described in terms of its specific embodiments, certain modifications and equivalents will be apparent to those skilled in the art and are intended to be included within the scope of the present invention.

### EXAMPLES

### Example 1: Preparation of Polymorphic Form D of Ertapenem Monosodium

Ertapenem sodium (50 g, HPLC Purity: 95%) was dissolved in aqueous sodium bicarbonate solution (9.3 g in 400 ml) at 0° to 5°C. Methanol (100 ml) was added to the solution and the pH of the solution was adjusted with a mixture of acetic acid and methanol 1 (1:1) to 5.5 to 5.7 at 0° to 5°C. The solution was diluted further with methanol (100 ml) and treated with activated carbon (5 g). The mixture was filtered and washed with a pre-cooled (0 to 5°C) mixture of water (100 ml) and methanol (50 ml). The combined filtrate was cooled to -5° to 0°C and n-propanol (250 ml) was added to the cooled solution, followed by the addition of seed ertapenem monosodium (0.5 g; obtained by following the present example without employing seed) at about -5°C. The mixture was stirred at -10° to -5°C for 15 to 20 minutes. A mixture of n-propanol (750 ml) and methanol (750 ml) was added slowly into the mixture in 90 to 100 minutes at -15° to -10°C and stirred for 2 to 3 h at -15° to -10°C. The mixture was filtered, washed with acetone (100 ml) and dried under vacuum for 30 minutes in nitrogen atmosphere to obtain the title compound (MW: 497.5) as a free flowing solid having an XRPD pattern as depicted in Figure 2 of the accompanied drawing.
Yield: 30 g
HPLC Purity: 98 %
Sodium Content: 4.3% w/w (by ISE method, on anhydrous basis)

### Example 2: Preparation of Sterile Carbapenem Antibiotic Composition

Polymorphic Form D of ertapenem monosodium (27.5 g) as prepared in Example 1 was added slowly to a solution of sodium bicarbonate (4.0 g) in water (100 ml) at 0° to 5°C with simultaneous addition of aqueous sodium hydroxide solution (10 % w/v) to maintain the pH of the solution between 7.4 and 7.7. The solution so obtained was filtered through a 0.2 micron membrane filter, loaded on a lyophilization tray in a tray lyophilizer and lyophilized according to the conditions provided below:

| **STEP** | **STEP TYPE** | **TEMPERATURE (°C)** | **VACUUM (mTorr)** | **TIME (h)** |
|---|---|---|---|---|
| 1 | Freezing | -40 | - | 2 |
| 2 | Freezing | -40 | - | 2 |
| 3 | Evacuation | - | 100 | - |
| 4 | Drying | -10 | 100 | 3 |
| 5 | Drying | 0 | 100 | 2 |
| 6 | Drying | 0 | 100 | 15 |
| 7 | Drying | 10 | 100 | 1 |
| 8 | Drying | 10 | 100 | 8 |
| 9 | Drying | 15 | 100 | 1 |
| 10 | Drying | 15 | 100 | 7 |
| 11 | Drying | 20 | 100 | 1 |
| 12 | Drying | 20 | 100 | 7 |
| 13 | Drying | 25 | 100 | 1 |
| 14 | Drying | 25 | 100 | 5 |
| Total Time | | | | 55 |

The sterile lyophilized pharmaceutical composition of the sodium salt of the compound of Formula II (Molecular Weight: 585.5) having an XRPD pattern as depicted in Figure 1 of the accompanied drawing was unloaded from the lyophilization tray.
Yield: 27.7 g
HPLC Purity: 97%
Dimer I and Dimer II: 0.70%
Dimer III: 0.2%
Dimer-H₂Oa and Dimer-H₂Ob: 0.45%
Dimer V: Not detectable
Ring Opened Impurity: 1.0%
Sodium Content: 11.0% w/w (by ISE method, on anhydrous basis)

## Claims

1. Polymorphic Form D of ertapenem monosodium having an XRPD pattern showing 2 theta values at 4.44±0.2, 5.26±0.2, 7.44±0.2, 8.12±0.2, 10.98±0.2, 12.74±0.2, 19.28±0.2, 22.93±0.2, 23.51±0.2, 25.07±0.2 and 30.15 ±0.2.

2. A process for the preparation of polymorphic Form D of ertapenem monosodium wherein the process comprises,
a) treating ertapenem monosodium with water and methanol to obtain a solution,
b) treating the solution obtained in step a) with n-propanol,
c) stirring the mixture obtained in step b) at a temperature of about 0°C or below to obtain a solid, and
d) treating the solid obtained in step c) with a solvent consisting of acetone to obtain polymorphic Form D of ertapenem monosodium.

3. A process according to claim 2, wherein the polymorphic Form D of ertapenem monosodium is further converted into a pharmaceutical composition comprising the compound of Formula II or its salts.
